# EUROPÄISCHE PATENTSCHRIFT

(11) **EP 1 747 609 B1**
(45) Veröffentlichungstag und Bekanntmachung des Hinweises auf die Patenterteilung: **29.08.2007**
(21) Anmeldenummer: 05732314.9
(22) Anmeldetag: 15.04.2005
(51) Int. Cl.: H02G 11/02, B65H 75/40

(54) **DREHÜBERTRAGER FÜR KABELVERBINDUNGEN**
ROTATION TRANSFORMER FOR CABLE CONNECTIONS
TRANSMETTEUR ROTATIF POUR DES RACCORDS DE CABLE

(30) Priorität: 11.05.2004 DE 202004007741 U
(43) Veröffentlichungstag der Anmeldung: 31.01.2007
(73) Patentinhaber: Wampfler Aktiengesellschaft, 79576 Weil am Rhein-Maerkt (DE)
(72) Erfinder: GÜNTHER, Wolfgang, 09322 Penig/Tauscha (DE)
(74) Vertreter: Gehrsitz, Stefan
(86) Internationale Anmeldenummer: PCT/EP2005/003967
(87) Internationale Veröffentlichungsnummer: WO 2005/112215

(56) Entgegenhaltungen:
- DE-A1- 4 026 782
- DE-A1- 19 928 731
- DE-B- 1 060 279
- DE-U- 7 030 622
- DE-U- 7 031 318
- US-A- 3 809 331

## Beschreibung

Die Erfindung betrifft einen Drehübertrager für eine Kabelverbindung nach dem Oberbegriff des Anspruchs 1.

Im Sinne dieser Beschreibung und der Ansprüche ist unter einem Kabel jedes beliebige längliche, flexible oder elastische Material zum Transport von Energie, Information oder Stoffen zu verstehen, insbesondere, aber nicht ausschließlich, also ein elektrisches Kabel, ein optisches (zum Beispiel Glasfasern aufweisendes) Kabel, eine Druckleitung etc.

Aus der JP 59.149276 ist eine Lösung bekannt, bei der das Kabel auf einer elastischen Spiralfeder befestigt ist und mit dem axialen Federende verdreht werden kann. Nachteilig bei dieser Lösung ist zum einen ihr großer Durchmesser und zum anderen, dass sich mit den Umdrehungen verändernde, in den Grenzlagen recht große Rückstellmoment des rotierenden Teils.

Aus der DE 402 6782 A1 ist eine Lösung bekannt, bei der ein schraubenförmig ausgebildeter Trägerkörper einen Wendepunkt aufweist und das Kabel stoffschlüssig in den Trägerkörper eingebettet und damit geführt ist.

Aus der DE 199 28 731 C2 ist eine Vorrichtung zum Auf-, Abwickeln und Aufbewahren von länglichen, flexiblen Teilen bekannt, die aber nicht als Drehübertrager ausgebildet ist. Diese Vorrichtung dient dem Zweck, wie bei einer Kabeltrommel ein Kabel aufzunehmen, welches durch Zug an den beiden Kabelenden aus der Trommel herausgezogen werden kann. Das Kabel weist hier jedoch keine Führung durch eine Lagerachse auf und aufgrund der nur zwei vorhandenen Räume zwischen benachbarten Trennscheiben ist eine kontrollierte Auf- und Abwicklung, wie sie für einen präzisen Drehübertrager benötigt wird, nicht möglich.

Die DE 703 0 622 und die DE 703 1 318 haben eine Vorrichtung zum Aufwickeln von elektrischen Messkabeln bzw. eine Kabel- und Schlauchkassette zum Gegenstand. Bei beiden Druckschriften wird das Problem der Drehübertragung aber in keiner Weise angesprochen, vielmehr befassen sich beide Schriften nur mit dem Speichern und Freigeben von Kabeln, das Problem der Verdrehung der Kabelenden gegeneinander spielt keine Rolle.

Die DE 1 060 279 B hat eine Schlauchtrommel für einen Vollschlauch zum Gegenstand, bei welcher der Anfang des Schlauches an der auf einer Hohlachse laufenden Nabe angeschlossen ist, wobei die gesamte Breite der Haspel durch Zwischenbleche in eine ungerade Anzahl von Schichten unterteilt ist und zwischen den jeweils einander zugekehrten Flächen der Bleche eine Stützblechspirale angeordnet ist, wobei die Zwischenbleche abwechselnd mit einem Ausschnitt oder mit einem Einschnitt versehen sind, der Einschnitt sektorartig abgewinkelt ist und die Stützblechspirale vom Fuß des Einschnitts bis zur unteren Kante des Ausschnitts verläuft.

Schließlich zeigt die DE 40 18 440 A1 eine spiralige Leitungsanordnung, die am einen Ende mit einer um eine Drehachse hin- und herdrehbaren Vorrichtung und am anderen Ende mit einer feststehenden Vorrichtung verbunden ist und mit einer Drehung der drehbaren Vorrichtung ein- oder ausgedreht wird. Der gegebene Lösungsvorschlag ist technisch außerordentlich kompliziert, da ein synchronisierender Antrieb der einzelnen Kaskaden notwendig ist.

Es besteht daher die Aufgabe, einen Drehübertrager für eine Kabelverbindung so auszubilden, dass er einfach und preisgünstig in der Herstellung ist, keine Rückstellmomente erzeugt, keinen besonderen Antrieb benötigt und keine speziellen Anforderungen an die Kabelgestaltung oder -einbettung stellt.

Gelöst wird diese Aufgabe mit den kennzeichnenden Merkmalen des Anspruchs 1. Vorteilhafte Ausgestaltungen sind den Unteransprüchen entnehmbar.

Ein Ausführungsbeispiel der Erfindung wird im folgenden unter Bezugnahme auf die einzelnen Zeichnungen näher erläutert. Hierbei zeigen:
- **Figur 1:**: Einen Querschnitt durch einen erfindungsgemäßen Drehübertrager;
- **Figur 2:**: Eine Draufsicht auf eine Kabelwicklung des in Figur 1 dargestellten Drehübertrages zwischen zwei Trennscheiben;
- **Figur 3:**: Eine Kabelwicklung des in Figur 1 dargestellten Drehübertrages zwischen dem nächsten Paar Trennscheiben;
- **Figur 4:**: Einen Querschnitt durch den in Figur 1 dargestellten Drehübertrager, wobei jede Trennscheibe gegenüber Figur 1 eine Umdrehung weitergedreht wurde;
- **Figur 5:**: Eine Darstellung gemäß Figur 2, wobei die Trennscheiben eine Umdrehung weitergedreht wurden;
- **Figur 6:**: Eine Darstellung gemäß Figur 3, wobei die Trennschreiben eine Umdrehung weitergedreht wurden;
- **Figur 7:**: Eine Draufsicht auf den Drehübertrager in Längsrichtung;
- **Figur 8:**: Einen Querschnitt durch einen Drehübertrager ohne Kabel unter Darstellung der Anschlaglaschen 5 gemäß Figur 7;
- **Figur 9:**: Eine Darstellung des Durchgangs des Kabels zwischen benachbarten Trennscheiben.

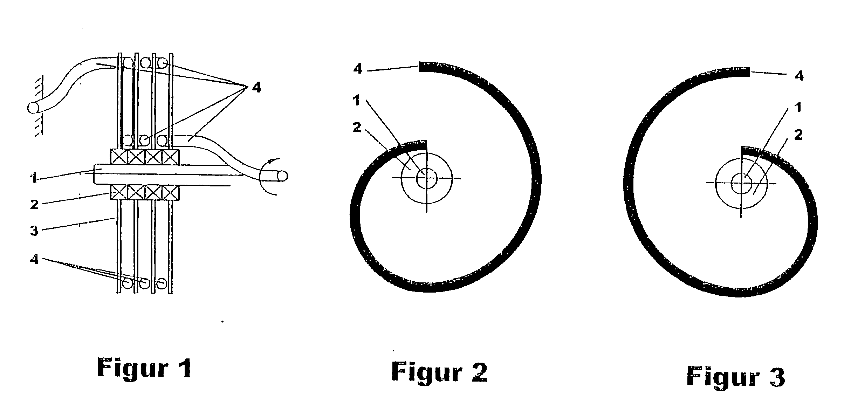

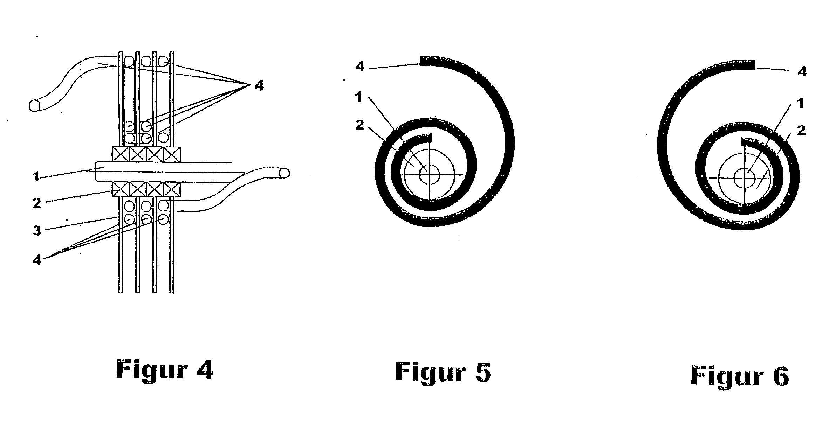

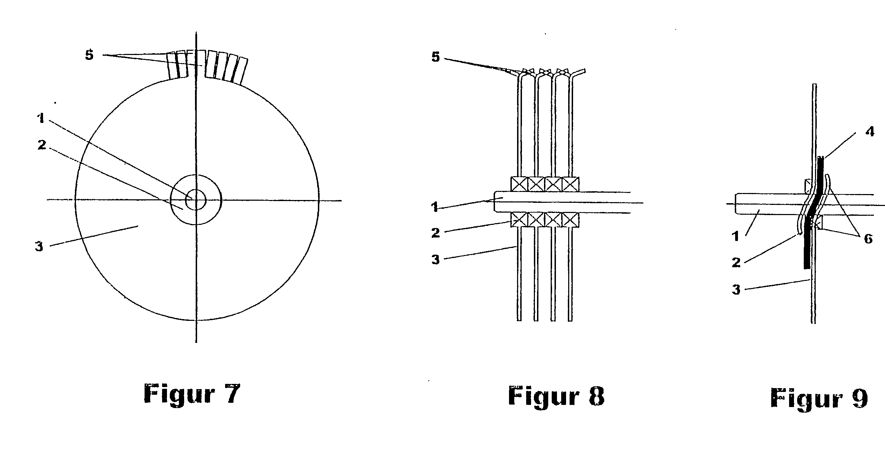

Figur 1 zeigt eine Längsachse 1, um welche die gesamte Anordnung drehbar ist. Auf der Achse 1 sitzen Lager 2, wobei jedes Lager 2 eine Trennscheibe 3, vorzugsweise aus Metall oder Kunststoff, trägt und die Trennscheiben 3 über die einzelne Lagerung auf den Lagern 2 unabhängig voneinander um die Lagerachse 1 rotieren können. Der Abstand der Trennscheiben 3 ist so bemessen, dass das zwischen ihnen liegende Kabel 4 ein geringes seitliches Spiel aufweist, so dass es ohne weiteres sich zwischen den Trennscheiben 3 bewegen kann und von diesen nicht festgeklemmt wird, aber daran gehindert wird, mehrere nebeneinander liegende Lagen zu bilden und auch kein nennenswertes seitliches Verrutschen aufeinanderfolgender Windungen stattfinden kann. Die Trennschreiben 3 weisen Durchbrüche auf, die im einzelnen in Figur 9 dargestellt sind, über die das Kabel 4 in die Räume zwischen benachbarten Trennscheiben 3 hindurchtreten kann. Diese Durchbrüche befinden sich sowohl innen als außen an den Trennscheiben 3, und das Kabel 4 ist in einem Raum zwischen zwei Trennscheiben zugeführt, dass es von innen nach außen spiralig verläuft, wie es in Figur 2 dargestellt ist. Daraufhin, tritt es durch den (nicht dargestellten) Durchbruch in der Trennscheibe 3 von außen in den Raum jenseits dieser Trennscheibe 3 ein und verläuft wiederum spiralig von außen nach innen. Diese Abwechslung des Wicklungsverlaufs erfolgt durch sämtliche Trennscheibenräume hindurch. Durch diese Kabelführung werden Kreuzungen des Kabels innerhalb eines Zwischenraumes zwischen benachbarten Trennscheiben 3 vermieden. Die Kabellänge der in den Figuren 2 und 3 dargestellten Bindung des Kabels 4 ist so bemessen, dass bei einer Umdrehung der Kabelzuführung je Zwischenraum gegenüber der Kabelabführung in den nächsten Zwischenraum gerade eine weitere Windung ermöglicht wird. Dieser Zustand ist in den ansonsten gleichen Figuren 4, 5 und 5 dargestellt. Hier ist jede Trennscheibe 3 gegenüber der vorherigen um eine Umdrehung weitergedreht worden.

Damit genau diese eine zusätzliche Umdrehung realisiert werden kann und das Kabel 4 nicht durch Zug zu stark belastet wird, besitzen die Trennscheiben 3, wie in Figur 7 dargestellt, am äußeren Umfang jeweils zwei Anschlaglaschen 5, welche wechselseitig axial gemäß Figur 8 verbogen sind. Damit ermöglichen die Anschlaglaschen 5 jeder Trennscheibe 3 genau eine Umdrehung Bewegungsfreiheit gegenüber der benachbarten Trennscheibe 3.

Weil durch jede einzelne Trennscheibe 3 etwa eine Umdrehung ermöglicht wird, ergibt sich eine Gesamtumdrehungszahl zwischen Kabelzuführung und -abführung des erfindungsgemäßen Drehübertragers, die gleich der Anzahl der Trennscheiben 3 (ohne Berücksichtigung der Gehäusebegrenzungen) ist.

In einer weiteren, nicht dargestellten Ausführungsvariante wird das Kabel 4 so zwischen den Trennscheiben 3 angeordnet, dass im zusammengewickelten Zustand 3 oder mehr Windungen entstehen. Die Relativverdrehung zwischen den benachbarten Trennscheiben wird dann durch einen elastischen Gleitstein und eine spiralige Führungsbahn mit entsprechendem Umdrehungswinkel begrenzt.

Wie oben erwähnt zeigt Figur 9, wie das Kabel 4 in den Trennscheiben 3 mittels Führungslaschen 6 fixiert und in der axialen Beweglichkeit gehalten wird, so dass die zwischen den Trennscheiben 3 liegende freie Länge konstant bleibt, ohne dass das Kabel geknickt wird.

## Patentansprüche

1. Drehübertrager für eine Kabelverbindung, wobei ein Ende eines Kabels (4) gegenüber seinem anderen Ende eine begrenzte Anzahl von Umdrehungen um eine Längsachse (1) unter Aufrechterhaltung einer ununterbrochenen Verbindung zwischen beiden Enden ausführen kann und die Längsachse (1) Trennscheiben (3) aufweist, **dadurch gekennzeichnet, dass** der Abstand der einzeln drehbar ausgebildeten Trennscheiben (3) so bemessen ist, dass maximal zwei Windungen des Kabels (4) zwischen zwei Trennscheiben (3) nur in einer Ebene liegen und das Kabel (4) dadurch kreuzungsfrei geführt ist, dass es zwischen den Trennscheibenpaaren (3) abwechselnd vom Außendurchmesser zur Längsachse (1) und umgekehrt gewunden ist.

2. Drehübertrager nach Anspruch 1, **dadurch gekennzeichnet, dass** die Trennscheiben (3) drehbar auf der Längsachse (1) gelagert sind und an ihrer Außenseite Anschlaglaschen (5) zur Begrenzung des maximalen Drehwinkels zweier benachbarter Trennscheiben (3) zueinander aufweisen.

3. Drehübertrager nach einem der voranstehenden Ansprüche, **dadurch gekennzeichnet, dass** die Anzahl der Umdrehungen der Enden des Kabels (4) durch die Anzahl der Trennscheiben (3) und die dort gelagerten Windungen bestimmt ist.

4. Drehübertrager nach einem der voranstehenden Ansprüche, **dadurch gekennzeichnet, dass** die Trennscheiben (3) sich zueinander um einen maximalen Winkel drehen können, der durch einen elastischen Gleitstein und eine spiralige Führungsbahn begrenzt wird.

5. Drehübertrager nach einem der voranstehenden Ansprüche, **dadurch gekennzeichnet, dass** die Trennscheiben (3) Durchbrüche mit Führungslaschen (6) aufweisen, welche das Kabel (4) gerade so stark fixieren, dass sich seine freie Länge zwischen benachbarten Trennscheiben (3) nicht verändern kann, es aber nicht durch zu starken Druck beschädigen oder beinträchtigen.

## Claims

1. Rotary connector for a cable connection, in which one end of a cable (4) is capable of executing a limited number of revolutions about a longitudinal axis (1) relative to its other end while an uninterrupted connection is maintained between the two ends, and the longitudinal axis (1) exhibits separating discs (3), **characterised in that** the distance between the individually rotatable separating discs (3) is such that a maximum of two turns of the cable (4) lie between two separating discs (3) in just one plane and the cable (4) is consequently guided without crossing **in that** it is wound between the pairs of separating discs (3) alternating from the outside diameter to the longitudinal axis (1) and vice versa.

2. Rotary connector according to claim 1, **characterised in that** the separating discs (3) are mounted rotatably on the longitudinal axis (1) and on their outside exhibit stops (5) for limiting the maximum angle of rotation of two neighbouring separating discs (3) relative to one another.

3. Rotary connector according to one of the preceding claims, **characterised in that** the number of revolutions of the ends of the cable (4) is determined by the number of separating discs (3) and the turns stored there.

4. Rotary connector according to one of the preceding claims, **characterised in that** the separating discs (3) can rotate relative to one another through a maximum angle which is limited by an elastic block and a helical guide track.

5. Rotary connector according to one of the preceding claims, **characterised in that** the separating discs (3) exhibit openings with guides (6) which secure the cable (4) just securely enough so that its free length between neighbouring separating discs (3) cannot change, but it cannot be damaged or impaired by excessive pressure.

## Revendications

1. Transmetteur rotatif pour raccordement de câbles, une extrémité d'un câble (4) pouvant effectuer par rapport à son autre extrémité un nombre limité de rotations autour d'un axe longitudinal (1) en conservant une liaison ininterrompue entre les deux extrémités et l'axe longitudinal (1) comprenant des disques de séparation (3), **caractérisé en ce que** la distance entre les disques de séparation (3) conçus de manière à pouvoir tourner individuellement étant dimensionnée de sorte qu'au maximum deux enroulements du câble (4) entre deux disques de séparation (3) ne se situe que dans un plan et le câble (4) est guidé sans croisement **en ce qu'**il est enroulé entre les paires de disques de séparation (3) en alternance du diamètre extérieur à l'axe longitudinal (1) et inversement.

2. Transmetteur rotatif selon la revendication 1, **caractérisé en ce que** les disques de séparation (3) sont logés de manière rotative sur l'axe longitudinal (1) et comprennent sur leur côté extérieur des pattes de butée (5) destinées à limiter l'angle rotatif maximal de deux disques de séparation (3) voisins l'un par rapport à l'autre.

3. Transmetteur rotatif selon l'une quelconque des revendications précédentes, **caractérisé en ce que** le nombre de rotations des extrémités du câble (4) est défini par le nombre des disques de séparation (3) et les enroulements logés à cet endroit.

4. Transmetteur rotatif selon l'une quelconque des revendications précédentes, **caractérisé en ce que** les disques de séparation (3) peuvent être amenés en rotation les uns par rapport aux autres suivant un angle maximal qui est limité par un coulisseau élastique et une bande de guidage en spirale.

5. Transmetteur rotatif selon l'une quelconque des revendications précédentes, **caractérisé en ce que** les disques de séparation (3) comprennent des passages dotés de pattes de guidage (6), lesquelles fixent justement le câble (4) si fortement que sa longueur libre entre les disques de séparation (3) voisins ne peut varier mais pas le détériorer ni l'altérer à cause d'une trop forte pression.
